# EUROPEAN PATENT SPECIFICATION

(11) **EP 1 008 579 B1**
(45) Date of publication and mention of the grant of the patent: **07.07.2004**
(21) Application number: 00102011.4
(22) Date of filing: 29.03.1997
(51) Int. Cl.: C07C 33/12, C11B 9/00, A61K 7/46

(54) **Cyclopentenebutanol derivatives**
Cyclopentanbutanolderivate
Dérivés de cyclopentanebutanol

(30) Priority: 09.04.1996 EP 96105555; 10.04.1996 EP 96105603
(43) Date of publication of application: 14.06.2000
(62) Divisional of application: 97105322.8
(73) Proprietor: Givaudan SA, 1214 Vernier-Genève (CH)
(72) Inventor: Bajgrowicz, Jerzy A., 8053 Zürich (CH); Frater, Georg, 8400 Winterthur (CH)
(74) Representative: McStea, John Anthony

(56) References cited:
- EP-A- 0 155 591
- WO-A-92/22518
- DD-A- 266 347
- US-A- 4 052 341
- K. SCHULZE, ET AL.: "Synthese, Struktur und Reaktionen von alpha-Campholenepoxiden" LIEBIGS ANNALEN DER CHEMIE, no. 9, September 1993 (1993-09), pages 987-991, XP002067555 Verlag Chemie, Weinheim, DE ISSN: 0170-2041
- K. SCHLUZE, ET AL.: "alpha-Methylierter Fencholen- un alpha-Campholenaldehyd - Synthese und Reaktionen" JOURNAL FUR PRAKTISCHE CHEMIE, CHEMIKER ZEITUNG, vol. 335, no. 8, August 1993 (1993-08), pages 687-693, XP002067556 Wiley VCH, Weinheim, DE ISSN: 1436-9966
- A.S. DIMOGLO, ET AL.: "Investigation of the relationship between sandalwood odour and chemical structure: electron-topological approach" NEW JOURNAL OF CHEMISTRY, vol. 19, no. 2, February 1995 (1995-02), pages 149-154, XP002067557 CNRS-Gauthier-Villars, Montrouge, FR ISSN: 1144-0546

## Description

The invention relates to novel odorants derived from campholenic aldehyde. In particular these are compounds of the general formula 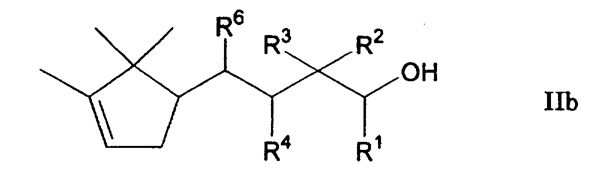 wherein R¹ to R³ and R⁶ are H, methyl or ethyl, R⁴ is methyl or ethyl, or R¹+R² form -(C)(H₂)ₙ-,with n being 3 or 4.

A possible route to these novel compounds starting from campholenic aldehyde is outlined in reaction scheme 1.

As shown in the scheme, the route from the known intermediates makes use of known chemical transformations. These are:
a) aldol condensation, effecting elongation of the side chain, e.g. by reacting campholenic aldehyde with the reactant R²CH₂C(O)R¹ under basic conditions, e.g. using any organic or inorganic base,
b) reduction of carbonyl groups to saturated and unsaturated alcohols, e.g. using hydrides, e.g. borohydrides, e.g. NaBH₄ in alkanols,
c) conjugated addition of organometallics to α,β-enones, leading to β-substituted carbonyl compounds,
   e.g. by the couple MeMgBr/CuI, conveniently in an ether as solvent,
d) reduction of α,β- or β,γ-enones to saturated alcohols,
   e.g. by catalytic hydrogenation, such as H₂/Pt, using any inert organic solvent,

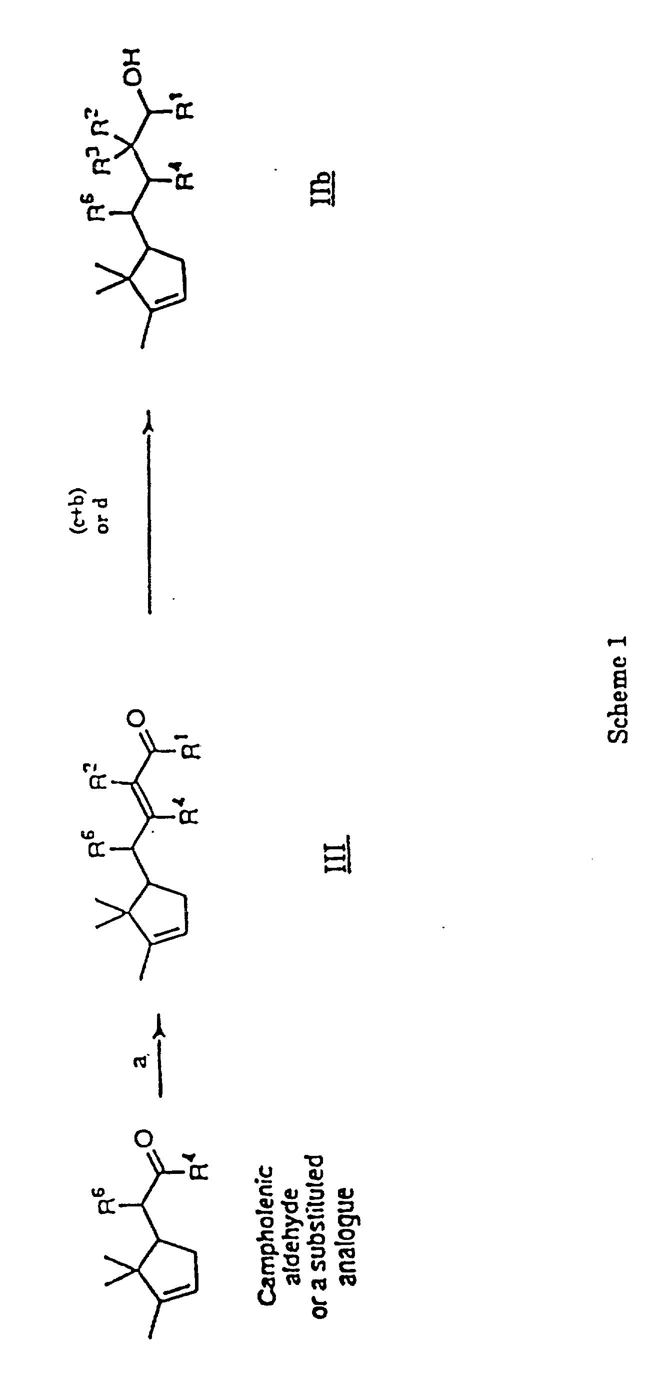

As pointed out above, these transformations as exemplified in the experimental part are known and their principles described in detail, e,g. in Comprehensive Organic Synthesis, Ed. Trost B.M., Fleming I., Pergamon Press, Oxford, England 1991, namely in
a) vol 2, p 133 seq.
b) vol 8, p 1 seq.
c) vol 4, p 69 seq.
d) vol 8, p 523 seq.

Campholenic aldehyde is a most important starting material for the synthesis of synthetic odorants exhibiting the odour profile of sandalwood oil (see, e.g. US patents 4052341, 4696766).

The new compounds of the general formula IIb (with R⁴=Me) exhibit useful olfactory properties, their odour belonging also to the amber/woody/ sandalwood family of odours.

The present invention thus comprises the compounds of the general formula IIb and their use as odorants.

The olfactory properties of the novel compounds harmonize with a multitude of natural or synthetic products widely used in compositions, in particular for generating middle and bottom notes, since the novel compounds are endowed with very good tenacity.

The compounds harmonize particularly well with all floral notes, in particular with rose, iris, jasmine, ylang-ylang and narcissus notes. They also harmonize with balsamic or resinous dry-out notes such as styrax, incense, and benzoin, and woody notes, such as oak moss or tree moss, patchouli and vetiver.

They thus provide most distinguished mixtures with a multitude of natural and synthetic raw materials.

Examples are:
- natural products, such as, for example, tree moss absolute, basil oil, tropical fruit oils (such as bergamot oil, mandarine oil, etc.), mastix absolute, myrtle oil, palmarosa oil, patchouli oil, petitgrain oil, wormwood oil, lavender oil, rose oil, jasmin oil or ylang-ylang oil etc.;
- alcohols, such as farnesol, geraniol, linalool, nerol, phenylethyl alcohol, rhodinol, cinnamic alcohol, cis-3-hexenol, menthol, α-terpineol etc.;
- aldehydes, such as citral, α-hexyl cinnamaldehyde, hydroxycitronellal, Lilial, (p-tert-butyl-α-methyl-dihydrocinnamaldehyde), methylnonylacetaldehyde, phenylacetaldehyde, anisaldehyde, vanillin etc.;
- ketones, such as allylionone, α-ionone, β-ionone, isoraldein (isomethyl-α-ionone), verbenone, nootkatone, geranylacetone etc.;
- esters, such as allyl phenoxyacetate, benzyl salicylate, cinnamyl propionate, citronellyl acetate, decyl acetate, dimethylbenzylcarbinyl acetate, ethyl acetoacetate, cis-3-hexenyl isobutyrate, linalyl acetate, methyl dihydrojasmonate, styrallyl acetate, vetiveryl acetate, benzyl acetate, cis-3-hexenyl salicylate, geranyl acetate etc.;
- lactones, such as γ-undecalactone, δ-decalactone, pentadecan-15-olide (Exaltolid), 12-oxahexadecanolide (Hibiscolide), etc.;
- acetals, such as Viridine (1,1-dimethoxy-2-phenylethane) etc.;
- various components often used in perfumery, such as indole, p-menthane-8-thiol-3-one, methyleugenol, eugenol, anethol etc.

The percentages in which the new compounds are used may vary within wide limits ranging from a few parts per thousand in mass market products (e.g. cleaning, deodorant) up to a few per cent in alcoholic extracts for (fine) perfumery. "Overdoses" of up to 20% of these derivatives come also in consideration, and may thus impart very particular effects e.g. in combination with synthetic musks. However, even small amounts of the novel compounds provide the odorant compositions with a rich sandalwood or ambery/woody effect and increase the volume (strength and diffusivity) and substantivity of their odour.

There is really no restriction regarding the type of formulations and the destination of the actual finished product: thus, eau de cologne, toilet water, scented water, perfume, cream, shampoo, deodorant, soap, detergent powder, household cleaner, softener, etc. come into consideration.

The compounds integrate into a multitude of compositions, e.g. oriental chypres, green and woody, floral leathers, fougère tobaccos and fruity aldehydes, etc. They provide, via their olfactory note, exceptional richness and linkage between the dry-out constituents of the compositions by providing more volume, warmth and roundness and augmenting sandalwood and woody aspects.

The campholenic aldehyde derivatives used as intermediates in the following examples were obtained starting from a ∼1:2 mixture of (S)-(―) and (R)-(+) campholenic aldehyde, both enantiomers being available from the suitable α-pinene. However, the general formula IIb should encompass both the pure isomers and mixtures of configurational, namely opticals isomers as, since all these isomers can be generated using the appropriate starting materials and synthetic methods.

The structures of the compounds described in the example has been proven by their IR, NMR and mass spectra. All compounds are colourless oils.

### Example 1

### 2,3-Dimethyl-4-(1,2,2-trimethylcyclopent-3-enyl)butan-1-ol

### a) 2,3-dimethyl-4-(2,2,3-trimethylcyclopent-3-enyl)butanal

100 ml (0.30 mol) of methylmagnesium bromide solution in diethyl ether was added to 60.0 g (0.32 mol) of cuprous iodide suspended in 350 ml of the same solvent at -10°C, followed by addition of 52.0 g (0.27 mol) of 2-methyl-4-(2,2,3-trimethylcyclopent-3-enyl)but-2-enal dissolved in 300 ml of anhydrous diethyl ether at 0°C and stirring at the same temperature was continued for 0.5 hours. The reaction mixture was treated with 200 ml of 1.0 N hydrochloric acid, decanted and the organic layer was washed with 2 x 300 ml of brine, dried (MgSO₄) and concentrated in vacuo. The residue was purified by flash chromatography on silica gel (eluent: hexane/MTBE 15:1) to give 28.2 g (50% yield) of 2,3-dimethyl-4-(2,2,3-trimethylcyclopent-3-enyl)butanal.
IR (neat): 3036, 2957, 2930, 2874, 2834, 2700, 1725, 1460, 1383, 1360, 1015, 798 cm⁻¹.

### b) 2,3-dimethyl-4-(2,2,3-trimethylcyclopent-3-enyl)butan-1-ol

A solution of 18.0 g (86 mmol) of 2,3-dimethyl-4-(2,2,3-trimethylcyclopent-3-enyl)butanal in 50 ml of ethanol was added dropwise at 0°C to 45.0 g (0.11 mol) of sodium borohydride suspended in 200 ml of the same solvent. After 18 hours of stirring at room temperature 100 ml of 1.0 N aqueous hydrochloric acid was added dropwise at 0°C. The reaction mixture was extracted with 200 ml of MTBE, the extract washed with with 3 x 100 ml of brine, dried (MgSO₄) and concentrated in vacuo. The residue was distilled at 82-86°C/0.1 Torr to give 14.1 g (78% yield) of 2,3-dimethyl-4-(2,2,3-trimethylcyclopent-3-enyl)butan-1-ol.
IR (neat): 3338, 3037, 2956, 2928, 2834, 1461, 1381, 1360, 1024, 798 cm⁻¹. Odour: sandalwood, amber, fruity, floral.

## Claims

1. Compounds of the formula 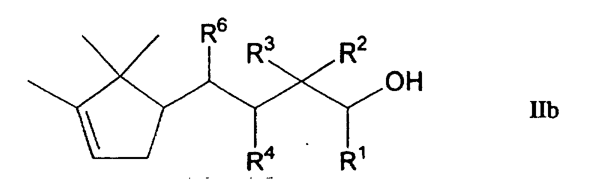 wherein R¹ to R³ and R⁶ are H, methyl or ethyl, R⁴ is methyl or ethyl, or R¹ + R² form -(CH₂)ₙ₋, with n being 3 or 4.

2. The compound 2,3-dimethyl-4-(2,2,3-trimethylcyclopent-3-enyl)butan-1-ol of formula IIb according claim 1.

3. Use of a compound of claim 1, especially of 2,3-dimethyl-4-(2,2,3-trimethylcyclopent-3-enyl)butan-1-ol, as an odorant.

4. An odorant composition containing at least one compound of formula IIb according to claim 1, especially a composition containing 2,3-dimethyl-4-(2,2,3-trimethylcyclopent-3-enyl)butan-1-ol.

## Patentansprüche

1. Verbindungen der Formel 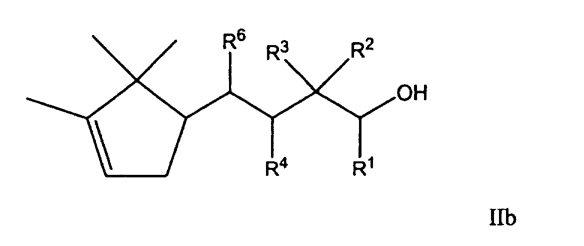 worin R¹ bis R³ und R⁶ H, Methyl oder Ethyl darstellen, R⁴ Methyl oder Ethyl ist, oder R¹ + R² ― (CH₂)ₙ ― bilden, wobei n 3 oder 4 ist.

2. Verbindung 2,3-Dimethyl-4-(2,2,3-trimethylcyclopent-3-enyl)butan-1-ol der Formel IIb nach Anspruch 1.

3. Verwendung einer Verbindung nach Anspruch 1 als Geruchsmittel, im Besonderen die Verwendung von 2,3-Dimethyl-4-(2,2,3-trimethylcyclopent-3-enyl)butan-1-ol.

4. Geruchsmittelzusammensetzung enthaltend mindestens eine Verbindung der Formel IIb gemäss Anspruch 1, im Besonderen eine Zusammensetzung 2;3-Dimethyl-4-(2,2,3-trimethylcyclopent-3-enyl)butan-1-ol enthaltend.

## Revendications

1. Composés de formule 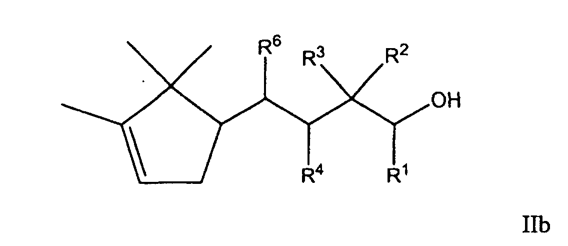 dans laquelle R¹ à R³ et R⁶ sont H, un groupe méthyle ou éthyle, R⁴ est un groupe méthyle ou éthyle, ou R¹ + R² representent ― (CH₂)ₙ-, n valant 3 ou 4.

2. Le composé 2,3-diméthyl-4-(2,2,3-triméthylcyclopent-3-enyl)butan-1-ol de la formule IIb selon la revendication 1.

3. Utilisation d'un composé selon la revendication 1, en particulier de 2,3-diméthyl-4-(2,2,3-triméthylcyclopent-3-enyl)butan-1-ol, comme matière odorante.

4. Composition odorante contenant au moins un composé de la formule IIb selon la revendication 1, en particulier une composition contenant 2,3-diméthyl-4-(2,2,3-triméthylcyclopent-3-enyl)butan-1-ol.
